# EUROPEAN PATENT APPLICATION

(11) **EP 4 269 557 A1**
(43) Date of publication of application: **01.11.2023**
(21) Application number: 21909910.8
(22) Date of filing: 14.10.2021
(51) Int. Cl.: C12M 3/00, C12M 1/00

(54) **CELL CULTURING VESSEL AND CELL CULTURING APPARATUS**

(30) Priority: 22.12.2020 JP 2020212308
(71) Applicant: Shimadzu Corporation, Nakagyo-ku, Kyoto-shi, Kyoto 604-8511 (JP)
(72) Inventor: INOUE, Tsunehiro, Kyoto-shi, Kyoto 604-8511 (JP); HASHIMOTO, Toyoyuki, Kyoto-shi, Kyoto 604-8511 (JP); YONEDA, Yasuko, Kyoto-shi, Kyoto 604-8511 (JP); TAKUBO, Kenji, Kyoto-shi, Kyoto 604-8511 (JP); FUJIYAMA, Yoichi, Kyoto-shi, Kyoto 604-8511 (JP); OHKUBO, Tomoki, Kyoto-shi, Kyoto 604-8511 (JP); OZEKI, Eiichi, Kyoto-shi, Kyoto 604-8511 (JP); TAKAI, Ryogo, Kyoto-shi, Kyoto 604-8511 (JP); GOTO, Hiroomi, Kyoto-shi, Kyoto 604-8511 (JP); TOMITA, Sadamu, Kyoto-shi, Kyoto 604-8511 (JP)
(74) Representative: Kilian Kilian & Partner mbB
(86) International application number: PCT/JP2021/038015
(87) International publication number: WO 2022/137752

(57) **Abstract**

A cell culture container includes: an insert member having a membrane on which a cell is seeded, the insert member defining a first inner space that functions as an anaerobic chamber; a container having an attachment/detachment portion to and from which the insert member is attachable and detachable, the container defining a second inner space that functions as an aerobic chamber; a sealing member that closes an opening of the aerobic chamber between the attachment/detachment portion and the insert member with the insert member being attached to the attachment/detachment portion; and a transfer mechanism that transfers force to the sealing member. The sealing member closes the opening in response to an input of the force from the transfer mechanism.

## Description

### TECHNICAL FIELD

The present invention relates to a cell culture container and a cell culture device.

### BACKGROUND ART

A cell culture container described in PTL 1 (WO 2018/079793) has a culture tank and a cell culture insert (hereinafter referred to as "cell culture insert"). The culture tank is provided with an opening that communicates with inside of the culture tank. The cell culture insert has a tubular portion and a porous membrane (hereinafter referred to as "membrane") that closes a lower end of the tubular portion. The tubular portion is inserted into the opening such that the membrane is located inside the culture tank. A medium is stored inside the culture tank.

### CITATION LIST

### PATENT LITERATURE

PTL 1: WO 2018/079793

### SUMMARY OF INVENTION

### TECHNICAL PROBLEM

When there is a clearance between the opening of the culture tank and the tubular portion of the cell culture insert (i.e., when the inner diameter of the opening is larger than the outer diameter of the tubular portion), a positive pressure is less likely to be applied to the membrane even when the tubular portion is inserted in the opening. However, in order to maintain airtightness inside the culture tank provided with the medium, it is necessary to seal the clearance with a sealing member. Further, in order to detach the cell culture insert from the culture tank, it is necessary to remove the sealing member.

It is an object of the present invention to provide a cell culture container to facilitate attachment and detachment of a cell culture insert while suppressing a positive pressure from being applied to a membrane of the cell culture insert.

### SOLUTION TO PROBLEM

A cell culture container of the present invention includes: an insert member having a membrane on which a cell is seeded, the insert member defining a first inner space that functions as an anaerobic chamber; a container having an attachment/detachment portion to and from which the insert member is attachable and detachable, the container defining a second inner space that functions as an aerobic chamber; a sealing member that closes an opening of the aerobic chamber between the attachment/detachment portion and the insert member with the insert member being attached to the attachment/detachment portion; and a transfer mechanism that transfers force to the sealing member. The sealing member closes the opening in response to an input of the force from the transfer mechanism.

### ADVANTAGEOUS EFFECTS OF INVENTION

According to the cell culture container of the present invention, attachment and detachment of the cell culture insert can be facilitated while suppressing a positive pressure from being applied to the membrane of the cell culture insert.

### BRIEF DESCRIPTION OF DRAWINGS

Fig. 1 is a cross sectional view of a cell culture container 100.
Fig. 2 is a plan view of a container main body 10.
Fig. 3 is a cross sectional view at III-III of Fig. 2.
Fig. 4 is a side view of container main body 10.
Fig. 5 is a plan view of a cell culture insert 20.
Fig. 6 is a cross sectional view at VI-VI of Fig. 5.
Fig. 7 is a plan view of a cover member 30.
Fig. 8 is a cross sectional view at VIII-VIII of Fig. 7.
Fig. 9 is a plan view of a cover member 60.
Fig. 10 is a cross sectional view at X-X of Fig. 9.
Fig. 11 is a cross sectional view of a cell culture container 200.
Fig. 12A is a first explanatory diagram showing a method of assembling cell culture container 200.
Fig. 12B is a second explanatory diagram showing the method of assembling cell culture container 200.
Fig. 12C is a third explanatory diagram showing the method of assembling cell culture container 200.
Fig. 13 is a cross sectional view of a cell culture container 200 according to a modification.
Fig. 14 is an explanatory diagram for flushing for cover member 30.
Fig. 15 is a schematic view of a cell culture device 300.

### DESCRIPTION OF EMBODIMENTS

Embodiments of the present invention will be described in detail with reference to figures. In the figures described below, the same or corresponding portions are denoted by the same reference characters and the same explanation will not be described repeatedly.

### (First Embodiment)

A cell culture container (hereinafter referred to as "cell culture container 100") according to a first embodiment will be described.

### <Configuration of Cell Culture Container According to First Embodiment>

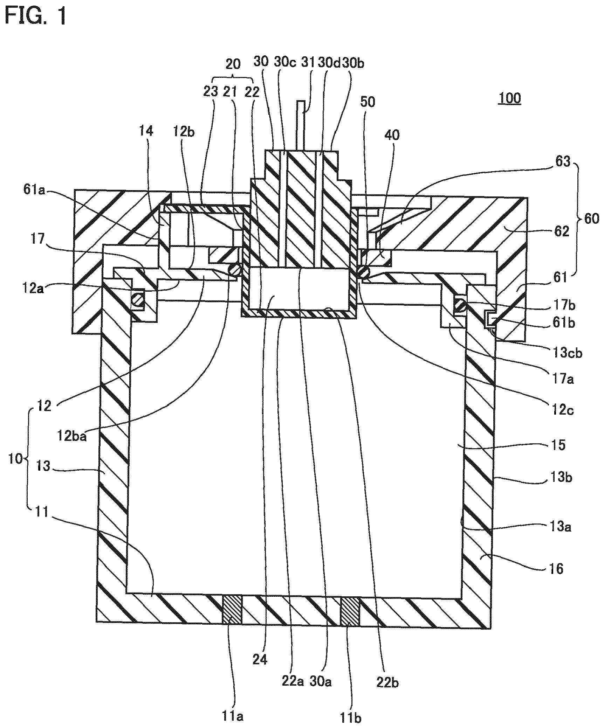

Fig. 1 is a cross sectional view of cell culture container 100. As shown in Fig. 1, cell culture container 100 has a container main body 10, a cell culture insert 20, a cover member 30, a loop-shaped member 40, an O-ring 50, and a cover member 60.

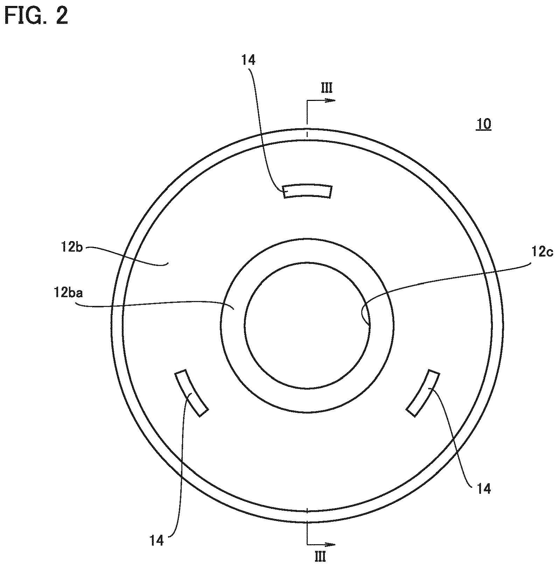

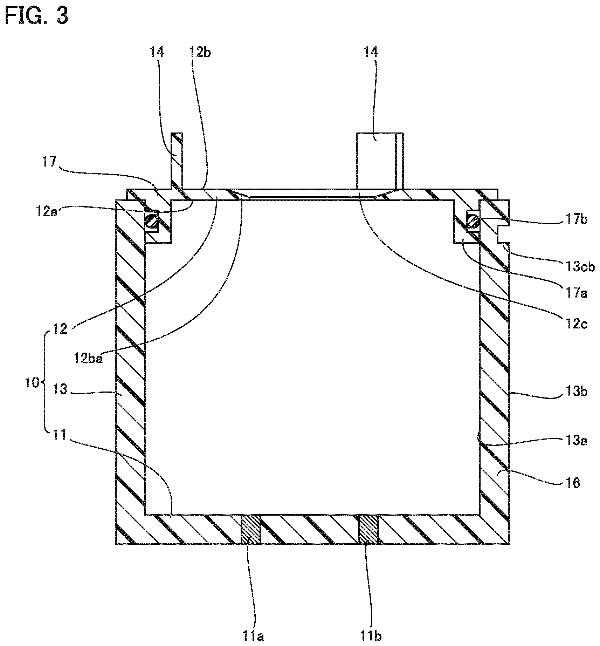

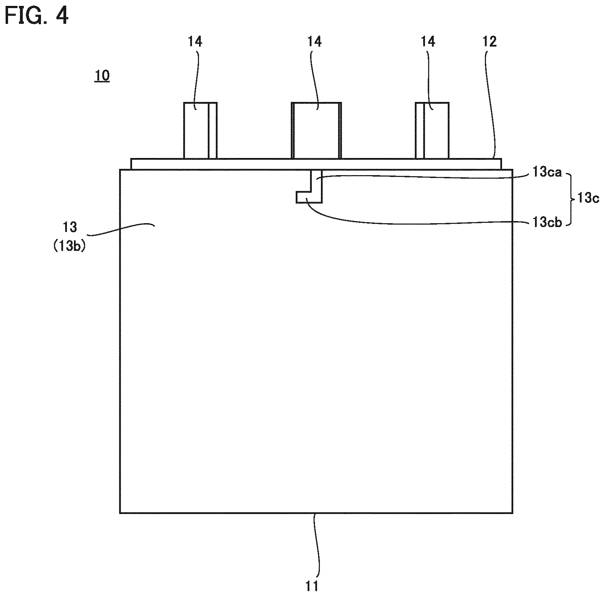

Fig. 2 is a plan view of container main body 10. Fig. 3 is a cross sectional view at III-III of Fig. 2. Fig. 4 is a side view of container main body 10. As shown in Figs. 2 to 4, container main body 10 has a bottom wall 11, an upper wall 12, and a side wall 13.

Bottom wall 11 and upper wall 12 face each other with a space being interposed therebetween. Side wall 13 is contiguous to bottom wall 11 and upper wall 12. An inner space of container main body 10 is defined by a bottom wall 11, an upper wall 12, and a side wall 13. A first medium 15 is stored in container main body 10 (see Fig. 1). An electrode 11a and an electrode 11b are embedded in bottom wall 11. Electrode 11a and electrode 11b are exposed to outside of container main body 10. Electrode 11a and electrode 11b are electrically connected to first medium 15.

Upper wall 12 has a first surface 12a and a second surface 12b. First surface 12a is a surface that faces bottom wall 11. Second surface 12b is a surface opposite to first surface 12a. A through hole 12c is formed in upper wall 12. Through hole 12c extends through upper wall 12 in a thickness direction and communicates with the inner space of container main body 10. Through hole 12c is disposed at the center of upper wall 12 when viewed in a plan view.

Second surface 12b has an inclination surface 12ba. Inclination surface 12ba surrounds through hole 12c when viewed in a plan view. That is, inclination surface 12ba has a loop shape when viewed in a plan view. Inclination surface 12ba is inclined such that a distance between inclination surface 12ba and first surface 12a is smaller in a direction toward through hole 12c.

Upper wall 12 has a standing wall 14. A plurality of standing walls 14 are provided. The number of standing walls 14 is, for example, three. Each of standing walls 14 protrudes from second surface 12b along a direction from bottom wall 11 toward upper wall 12. Standing walls 14 are disposed at equal intervals along a peripheral direction (along a circumference around the center of through hole 12c) when viewed in a plan view.

Side wall 13 has an annular shape when viewed in a cross sectional view orthogonal to the direction from bottom wall 11 toward upper wall 12. Side wall 13 has an inner wall surface 13a and an outer wall surface 13b. A groove 13c is formed in outer wall surface 13b. Outer wall surface 13b is depressed to the inner wall surface 13a side in groove 13c.

Groove 13c has a first portion 13ca and a second portion 13cb. First portion 13ca extends from an end of an upper end (end on the upper wall 12 side) of side wall 13 toward a lower end (end on the bottom wall 11 side) of side wall 13. Second portion 13cb extends from the lower end of first portion 13ca along the peripheral direction (along the circumference around the central axis of side wall 13).

Container main body 10 is constituted of, for example, a first member 16 and a second member 17. First member 16 is constituted of bottom wall 11 and side wall 13, and second member 17 is constituted of upper wall 12. Second member 17 has an insertion portion 17a inserted in side wall 13. An O-ring 17b airtightly seals between insertion portion 17a and side wall 13 (inner wall surface 13a).

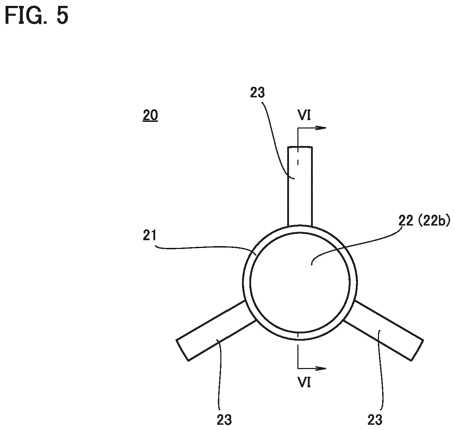

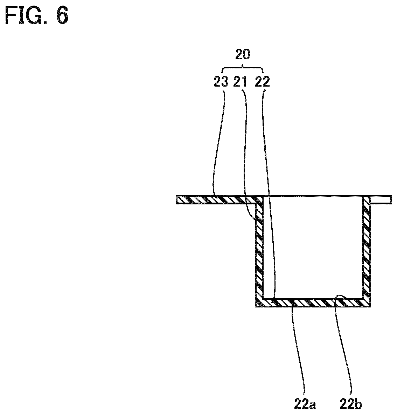

Fig. 5 is a plan view of cell culture insert 20. Fig. 6 is a cross sectional view at VI-VI of Fig. 5. As shown in Figs. 5 and 6, cell culture insert 20 has a tubular portion 21, a membrane 22, and a flange portion 23.

Tubular portion 21 has a tubular shape. When viewed in a cross sectional view orthogonal to a direction from an upper end to a lower end of tubular portion 21, tubular portion 21 has, for example, an annular shape. Tubular portion 21 is inserted in through hole 12c such that the lower end thereof is located inside container main body 10 (i.e., such that membrane 22 is located inside container main body 10) (see Fig. 1). There is a clearance between the outer peripheral surface of tubular portion 21 and the inner peripheral surface of through hole 12c (see Fig. 1).

A second medium 24 is stored in tubular portion 21 (see Fig. 1). A dissolved oxygen concentration in second medium 24 is lower than a dissolved oxygen concentration in first medium 15. That is, first medium 15 is an aerobic medium, and second medium 24 is an anaerobic medium. Second medium 24 may include bacteria (for example, anaerobic bacteria).

The lower end of tubular portion 21 is closed by membrane 22. Membrane 22 has a first surface 22a and a second surface 22b. First surface 22a is a surface that faces the inner side of container main body 10. Second surface 22b is a surface that faces the inner side of tubular portion 21 and that is opposite to first surface 22a. From another viewpoint, it can be said that part of the inner space of container main body 10 is defined by first surface 22a and part of the inner space of tubular portion 21 is defined by second surface 22b.

Cells are cultured (seeded) on second surface 22b. Each of the cells is, for example, an intestinal epithelial cell that forms a tight junction on second surface 22b. Specific examples of the cell include a Caco-2 cell. Membrane 22 is an oxygenpermeable membrane. Membrane 22 is, for example, a track-etched membrane composed of polycarbonate. Oxygen in first medium 15 is supplied, via membrane 22, to the cells cultured on second surface 22b.

Flange portion 23 protrudes from the outer peripheral surface of tubular portion 21 on the upper end side of tubular portion 21. A plurality of flange portions 23 are provided. The number of flange portions 23 is equal to, for example, the number of standing walls 14. Flange portions 23 are disposed at equal intervals along the peripheral direction (along the circumference around the central axis of tubular portion 21) when viewed in a plan view.

Flange portions 23 are disposed to overlap with standing walls 14 when viewed in a plan view, and are accordingly supported by standing walls 14. Movement of cell culture insert 20 in the direction from upper wall 12 toward bottom wall 11 is regulated by flange portions 23 being supported by standing walls 14.

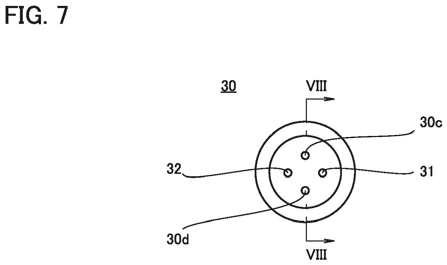

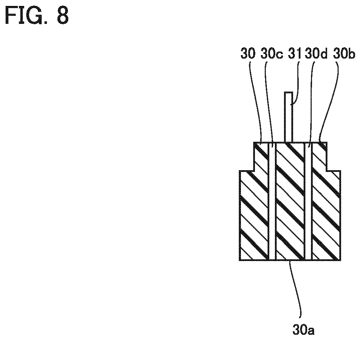

Cover member 30 is inserted into tubular portion 21 from the upper end side of tubular portion 21 (see Fig. 1). Fig. 7 is a plan view of cover member 30. Fig. 8 is a cross sectional view at VIII-VIII of Fig. 7. As shown in Figs. 7 and 8, cover member 30 has a first surface 30a and a second surface 30b. First surface 30a is a surface that faces the inner side of tubular portion 21. Second surface 30b is a surface opposite to first surface 30a. Cover member 30 is composed of a material having flexibility. Cover member 30 is composed of, for example, a silicone rubber.

A through hole 30c, a through hole 30d, a through hole 30e, and a through hole 30f are formed in cover member 30. Through hole 30c, through hole 30d, through hole 30e, and through hole 30f extend through cover member 30 along a direction from second surface 30b toward first surface 30a, and communicate with the inner space of tubular portion 21. An electrode 31 and an electrode 32 are inserted in through hole 30c and through hole 30d, respectively. Electrode 31 and electrode 32 are electrically connected to second medium 24.

As shown in Fig. 1, loop-shaped member 40 has a loop shape (for example, an annular shape). Tubular portion 21 is inserted into loop-shaped member 40. Loop-shaped member 40 is located above inclination surface 12ba.

O-ring 50 has a loop shape (for example, an annular shape). Tubular portion 21 is inserted in O-ring 50. O-ring 50 is located at a position close to the lower end of tubular portion 21 with respect to loop-shaped member 40. O-ring 50 is sandwiched between inclination surface 12ba and loop-shaped member 40. Thus, the inner space of container main body 10 is airtightly sealed.

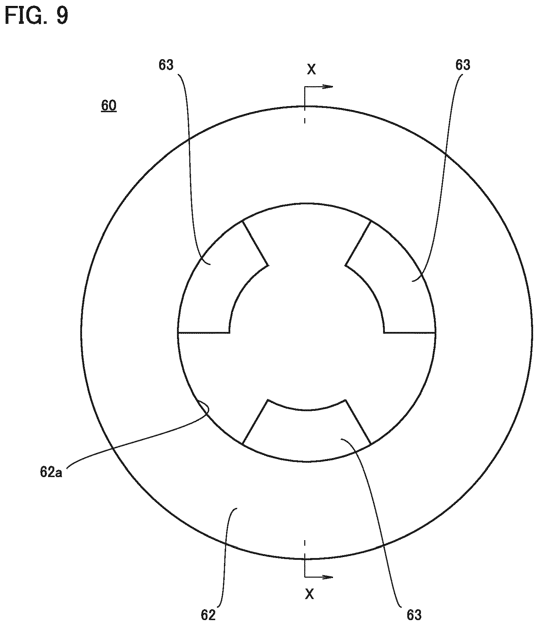

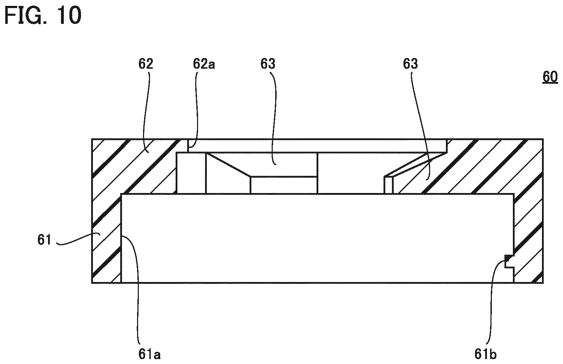

Fig. 9 is a plan view of cover member 60. Fig. 10 is a cross sectional view at X-X of Fig. 9. As shown in Figs. 9 and 10, cover member 60 has, for example, a circular shape when viewed in a plan view. Cover member 60 has a side wall 61 and an upper wall 62. Side wall 61 has an inner wall surface 61a. Upper wall 62 is contiguous to the upper end of side wall 61. A through hole 62a is formed in upper wall 62. Through hole 62a extends through upper wall 62 along the thickness direction. With cover member 60 being attached to container main body 10, the second surface 30b side of cover member 30 is exposed from through hole 62a.

Cover member 60 has a claw portion 63. Claw portion 63 protrudes from inner wall surface 61a along a radial direction (direction orthogonal to the central axis of side wall 61). A plurality of claw portions 63 are provided. Claw portions 63 are disposed at equal intervals along the peripheral direction (along the circumference around the central axis of side wall 61) when viewed in a plan view. Claw portions 63 are disposed at positions not overlapping with flange portions 23 and standing walls 14 when viewed in a plan view.

With cover member 60 being attached to container main body 10, claw portion 63 is in contact with loop-shaped member 40 (see Fig. 1). That is, loop-shaped member 40 is pressed against O-ring 50 along the direction from upper wall 12 toward bottom wall 11. Thus, O-ring 50 receives a reaction force from inclination surface 12ba and is accordingly deformed to airtightly seal the inner space of container main body 10.

A protrusion 61b is provided at inner wall surface 61a located at the lower end of side wall 61. Protrusion 61b is inserted in groove 13c (specifically, second portion 13cb). Thus, cover member 60 is attached to container main body 10.

### <Method of Assembling Cell Culture Container According to First Embodiment>

In assembling cell culture container 100, first, container main body 10 is prepared. Second, tubular portion 21 is inserted into loop-shaped member 40 and O-ring 50. On this occasion, O-ring 50 is located at a position close to the lower end of tubular portion 21 with respect to loop-shaped member 40. Third, tubular portion 21 is inserted into through hole 12c. On this occasion, cell culture insert 20 is aligned such that flange portions 23 are supported by standing walls 14 when viewed in a plan view. Fourth, cover member 60 is attached to container main body 10.

In attaching cover member 60, first, cover member 60 is aligned to insert protrusion 61b into the upper end side of first portion 13ca. Second, cover member 60 is pressed down in the direction from upper wall 12 toward bottom wall 11 until protrusion 61b reaches the lower end of first portion 13ca. Thus, claw portions 63 are brought into contact with loop-shaped member 40, and is pressed against O-ring 50 along the direction from upper wall 12 toward bottom wall 11. Third, by rotating cover member 60 around the central axis of side wall 61, protrusion 61b is inserted into second portion 13cb. In the manner described above, cover member 60 is attached to container main body 10.

It should be noted that when disassembling cell culture container 100, a procedure reverse to the above-described procedure of assembling may be performed.

### <Effects of Cell Culture Container According to First Embodiment>

In cell culture container 100, the inside of container main body 10 can be airtightly sealed by loop-shaped member 40 pressing O-ring 50 against inclination surface 12ba. Further, in cell culture container 100, by releasing the pressing of loop-shaped member 40 against O-ring 50, cell culture insert 20 can be detached from container main body 10. Thus, according to cell culture container 100, the cell culture insert can be readily attached and detached.

Since there is a clearance between the outer peripheral surface of tubular portion 21 and the inner peripheral surface of through hole 12c in cell culture container 100, a positive pressure is less likely to be applied to membrane 22 by inserting tubular portion 21 into through hole 12c.

Further, since movement of cell culture insert 20 in the direction from upper wall 12 toward bottom wall 11 is regulated in cell culture container 100, cell culture insert 20 is less likely to be moved in response to loop-shaped member 40 being pressed against O-ring 50. As a result, a positive pressure is less likely to be applied to membrane 22 even when airtightly sealing the inside of container main body 10.

Thus, according to cell culture container 100, attachment and detachment of cell culture insert 20 can be facilitated while suppressing a positive pressure from being applied to membrane 22.

### (Second Embodiment)

A cell culture container (hereinafter referred to as "cell culture container 200") according to a second embodiment will be described. Here, differences from cell culture container 100 will be mainly described and the same explanation will not be described repeatedly.

### <Configuration of Cell Culture Container According to Second Embodiment>

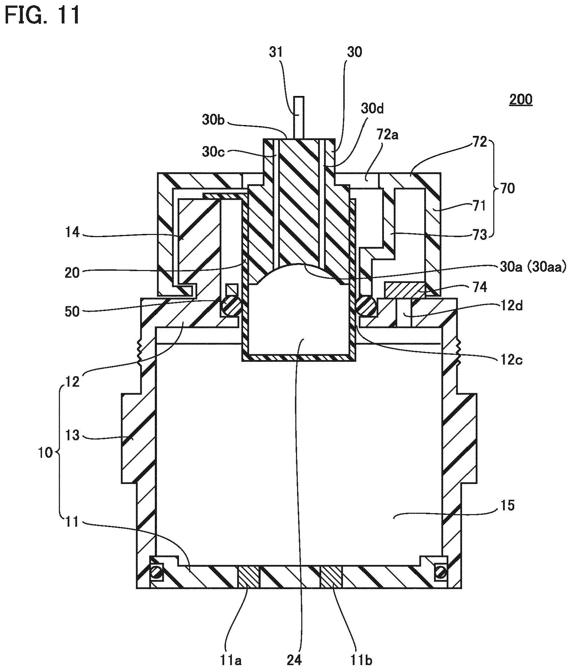

Fig. 11 is a cross sectional view of cell culture container 200. As shown in Fig. 11, cell culture container 200 has container main body 10, cell culture insert 20, cover member 30, and O-ring 50. Regarding this point, the configuration of cell culture container 200 is the same as the configuration of cell culture container 100.

Cell culture container 200 does not have loop-shaped member 40. Cell culture container 200 has a cover member 70 instead of cover member 60. A first surface 30a of cover member 30 is provided with a recess 30aa that is depressed to the second surface 30b side. A through hole 12d is formed in upper wall 12. No inclination surface 12ba is formed around through hole 12c. Regarding these points, the configuration of cell culture container 200 is different from the configuration of cell culture container 100. However, inclination surface 12ba may be formed around through hole 12c.

Cover member 70 has a side wall 71, an upper wall 72, and a pressure application portion 73. Cover member 70 is attached to container main body 10. More specifically, cover member 70 is anchored to standing walls 14 on the lower end side of side wall 71, and is accordingly attached to container main body 10.

Upper wall 72 is contiguous to the upper end of side wall 71. A through hole 72a is formed in upper wall 72. Via through hole 72a, cover member 30 can be inserted into cell culture insert 20.

Pressure application portion 73 is provided at the inner wall surface of upper wall 72. Pressure application portion 73 extends toward O-ring 50. With which cover member 70 being attached to container main body 10, pressure application portion 73 presses O-ring 50 toward upper wall 12. Thus, the clearance between through hole 12c and cell culture insert 20 is closed.

Through hole 12d communicates with the inner space of container main body 10. The inner diameter of through hole 12d is set to allow insertion of a tip of a pipette tip. The inner diameter of through hole 12d is, for example, 1 mm or more. A plurality of through holes 12d are provided. However, the number of through holes 12d may be one or may be three or more.

Cover member 70 is provided with an elastic member 74. Elastic member 74 is provided inside cover member 70. More specifically, elastic member 74 is provided on the lower end side of the inner wall surface of side wall 71. Elastic member 74 is disposed to close through hole 12d with cover member 70 being attached to container main body 10.

### <Method of Assembling Cell Culture Container According to First Embodiment>

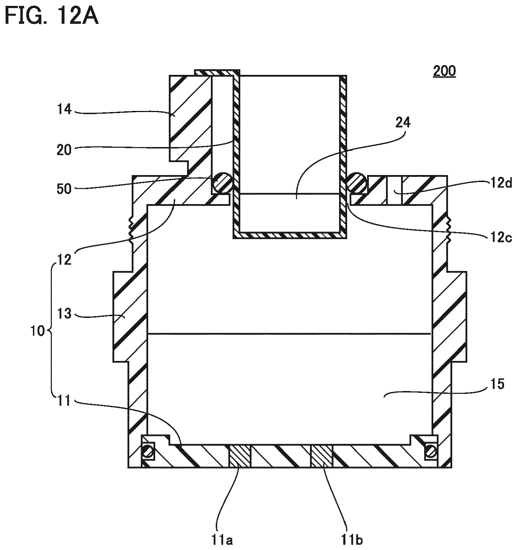

Fig. 12A is a first explanatory diagram showing a method of assembling cell culture container 200. As shown in Fig. 12A, in assembling cell culture container 200, first, cell culture insert 20 is inserted from through hole 12c into container main body 10 in which first medium 15 is introduced to such an extent that cell culture insert 20 is not in contact with first medium 15.

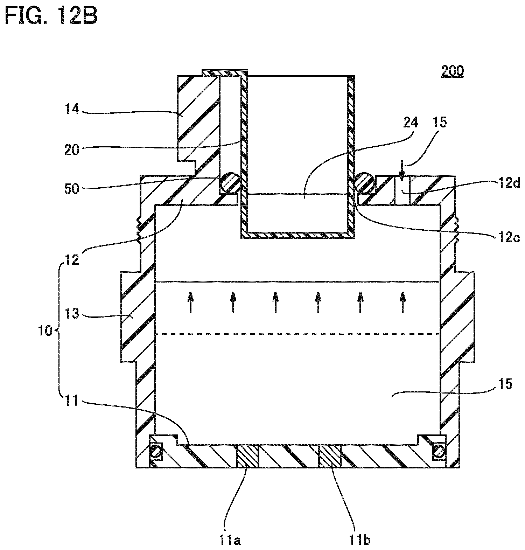

Fig. 12B is a second explanatory diagram showing the method of assembling cell culture container 200. As shown in Fig. 12B, in assembling cell culture container 200, second, first medium 15 is added from one of through holes 12d into container main body 10 using a pipette tip or the like. On this occasion, air in container main body 10 exits to outside via the other through hole 12d.

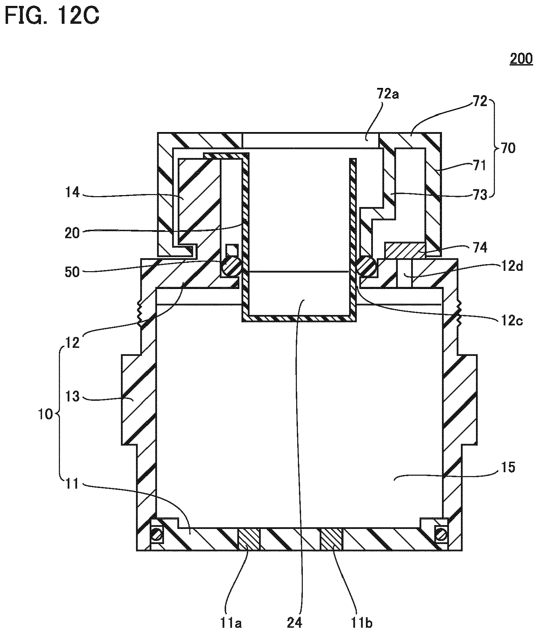

Fig. 12C is a third explanatory diagram showing the method of assembling cell culture container 200. As shown in Fig. 12C, in assembling cell culture container 200, third, cover member 70 is attached to container main body 10. Thus, O-ring 50 is pressed toward upper wall 12 by pressure application portion 73, thereby closing the clearance between through hole 12c and cell culture insert 20.

Then, cover member 30 is inserted into the inside of cell culture insert 20, thereby completing the assembling of cell culture container 200 having the structure shown in Fig. 11. It should be noted that when dissembling cell culture container 200, a procedure reverse to the procedure described above may be performed.

### <Effect of Cell Culture Container According to First Embodiment>

When assembling cell culture container 200, cell culture insert 20 is inserted into container main body 10 with a small amount of first medium 15 being stored in container main body 10, and then first medium 15 is replenished into container main body 10 via through hole 12d. Therefore, a positive pressure is less likely to be applied to membrane 22 when inserting cell culture insert 20. It should be noted that since through hole 12d is closed by elastic member 74 in a state after cover member 70 is attached, airtightness inside container main body 10 is ensured.

Further, in cell culture container 200, by attaching cover member 70 to container main body 10, pressure application portion 73 presses O-ring 50 against upper wall 12, thereby closing the clearance between through hole 12c and cell culture insert 20. Therefore, with cover member 70 being removed from container main body 10, attachment and detachment of cell culture insert 20 to and from container main body 10 can be facilitated.

Thus, according to cell culture container 200, attachment and detachment of cell culture insert 20 can be facilitated while suppressing a positive pressure from being applied to membrane 22.

### <Modification>

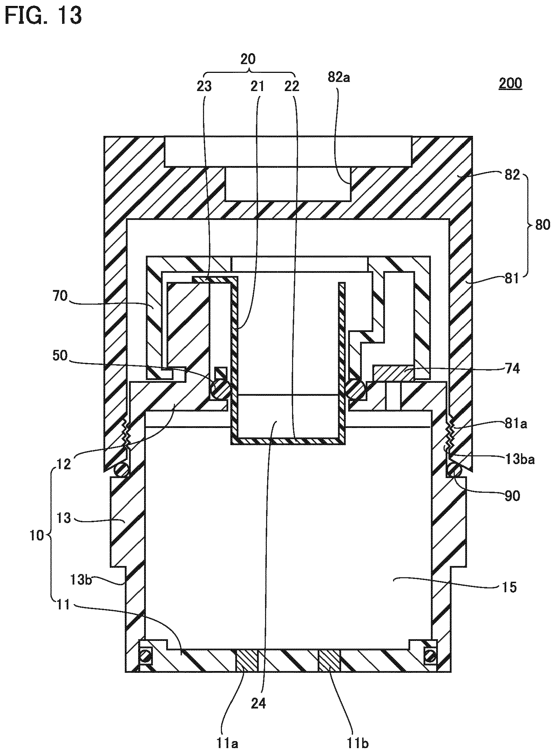

Fig. 13 is a cross sectional view of a cell culture container 200 according to a modification. As shown in Fig. 13, cell culture container 200 may further have a cover member 80 and an O-ring 90. Cover member 80 has a side wall 81 and an upper wall 82.

A thread groove 81a is formed in the inner wall surface of side wall 81. A thread 13ba is formed in outer wall surface 13b. Cover member 80 is attached to container main body 10 by screwing thread 13ba into thread groove 81a. This screwing is performed by rotating cover member 80 around the central axis relative to container main body 10. The central axis of cover member 80 is along a direction orthogonal to the main surfaces (first surface 22a and second surface 22b) of membrane 22.

Upper wall 82 is contiguous to the upper end of side wall 81. A recess 82a is formed in an outer wall surface of upper wall 82 (upper surface of cover member 80). An O-ring 90 airtightly seals between the inner wall surface of side wall 81 and the outer wall surface of side wall 13.

Cell culture container 200 shown in Figs. 12A to 12C is preferably assembled in an anaerobic chamber. Before performing this assembling, cell culture container 200 is placed in a chamber preceding the anaerobic chamber. The preceding chamber is under a decompression environment. When an influence of the decompression acts on membrane 22, a layer of cells seeded on membrane 22 may be damaged.

However, when cell culture container 200 has cover member 80, the inside of cover member 80 becomes a closed space, with the result that the influence of the decompression does not act on membrane 22. Thus, when cell culture container 200 has cover member 80, the layer of cells on membrane 22 can be suppressed from being damaged before assembling cell culture container 200 in the anaerobic chamber.

Before assembling cell culture container 200 shown in Figs. 12A to 12C, it is necessary to remove cover member 80 in the anaerobic chamber. This removal is performed inside the anaerobic chamber, which is an operation-restricted environment. However, since cover member 80 is attached to container main body 10 using the screw mechanism, cover member 80 can be readily removed even in the operation-restricted environment.

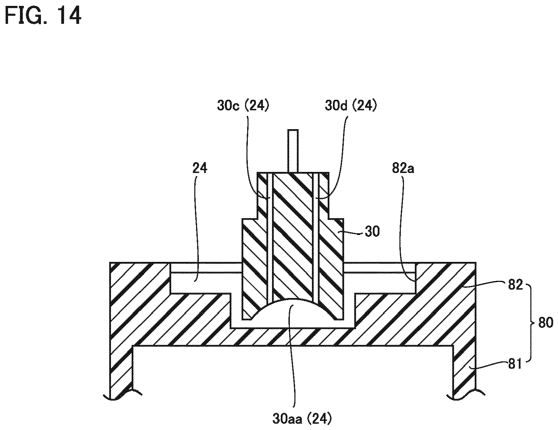

Recess 82a formed in the upper surface of cover member 80 can be used as a place in which cover member 30 is temporarily placed when assembling cell culture container 200. Fig. 14 is an explanatory diagram for flushing for cover member 30. The flushing for cover member 30 (that is, air inside recess 30aa, through hole 30c, and through hole 30d is replaced with second medium 24) is performed before cover member 30 is inserted into cell culture insert 20. As shown in Fig. 14, cover member 80 can be used readily in performing the flushing.

### (Configuration of Cell Culture Device According to Third Embodiment)

Hereinafter, a configuration of a cell culture device (hereinafter referred to as "cell culture device 300") according to a third embodiment will be described.

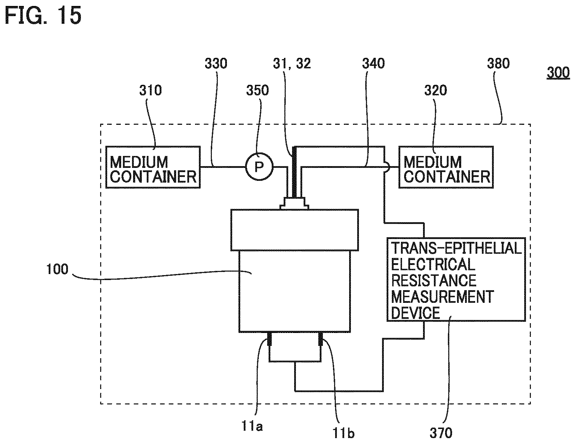

Fig. 15 is a schematic view of cell culture device 300. As shown in Fig. 15, cell culture device 300 has cell culture container 100, a medium container 310, a medium container 320, a tube 330, a tube 340, a pump 350, and a trans-epithelial electrical resistance measurement device 370. It should be noted that cell culture device 300 may have cell culture container 200 instead of cell culture container 100.

Cell culture container 100, medium container 310, medium container 320, tube 330, tube 340, pump 350, and trans-epithelial electrical resistance measurement device 370 are disposed inside an anaerobic chamber 380 (indicated by a dotted line in the figure).

Medium container 310 stores second medium 24 to be supplied to the inside of tubular portion 21, and medium container 320 stores second medium 24 discharged from the inside of tubular portion 21.

Tube 330 is connected to medium container 310 at one end and is connected to the inside of tubular portion 21 at the other end. The other end of tube 330 is inserted into through hole 30e (see Fig. 1). Tube 340 is connected to the inside of tubular portion 21 at one end, and is connected to medium container 320 at the other end. The other end of tube 340 is inserted in through hole 30f (see Fig. 1).

Pump 350 is disposed on a path of tube 330. Pump 350 is, for example, a tube pump.

By driving pump 350, second medium 24 stored in medium container 310 is supplied to the inside of tubular portion 21 through tube 330. Further, by driving pump 350, second medium 24 stored in tubular portion 21 is discharged to medium container 320 through tube 340. Thus, by driving pump 350, second medium 24 stored in tubular portion 21 is replaced.

Trans-epithelial electrical resistance measurement device 370 is electrically connected to electrode 11a and electrode 11b and is electrically connected to electrode 31 and electrode 32. Trans-epithelial electrical resistance measurement device 370 measures an electrical resistance value between electrodes 11a and 11b and electrodes 31 and 32 by, for example, a four-terminal method.

Since the electrical resistance value between electrodes 11a and 11b and electrodes 31 and 32 are changed depending on whether or not the cells cultured on second surface 22b form a tight junction, trans-epithelial electrical resistance measurement device 370 can be used to monitor the electrical resistance values so as to monitor whether or not the cells cultured on second surface 22b form a tight junction.

Although the embodiments of the present invention have been illustrated, the embodiments described above can be modified in various manners. Further, the scope of the present invention is not limited to the above-described embodiments. The scope of the present invention is defined by the terms of the claims, and is intended to include any modifications within the scope and meaning equivalent to the terms of the claims.

### REFERENCE SIGNS LIST

10: container main body; 11: bottom wall; 11a, 11b: electrode; 12: upper wall; 12a: first surface; 12b: second surface; 12ba: inclination surface; 12c: through hole; 13: side wall; 13a: inner wall surface; 13b: outer wall surface; 13ba: thread; 13c: groove; 13ca: first portion; 13cb: second portion; 13d: through hole; 14: standing wall; 15: first medium; 16: first member; 17: second member; 17a: insertion portion; 17b: O-ring; 20: cell culture insert; 21: tubular portion; 22: membrane; 22a: first surface; 22b: second surface; 23: flange portion; 24: second medium; 30: cover member; 30a: first surface; 30aa: recess; 30b: second surface; 30c, 30d, 30e, 30f: through hole; 31, 32: electrode; 40: loop-shaped member; 50: O-ring; 60: cover member; 61: side wall; 61a: inner wall; 61b: protrusion; 62: upper wall; 62a: through hole; 63: claw portion; 70: cover member; 71: side wall; 72: upper wall; 72a: through hole; 73: pressure application portion; 74: elastic member; 80: cover member; 81: side wall; 81a: thread groove; 82: upper wall; 82a: recess; 90: O-ring; 100, 200: cell culture container; 300: cell culture device; 310, 320: medium container; 330, 340: tube; 350: pump; 370: trans-epithelial electrical resistance measurement device; 380: anaerobic chamber.

## Claims

1. A cell culture container comprising:
an insert member having a membrane on which a cell is seeded, the insert member defining a first inner space that functions as an anaerobic chamber;
a container having an attachment/detachment portion to and from which the insert member is attachable and detachable, the container defining a second inner space that functions as an aerobic chamber;
a sealing member that closes an opening of the aerobic chamber between the attachment/detachment portion and the insert member with the insert member being attached to the attachment/detachment portion; and
a transfer mechanism that transfers force to the sealing member, wherein
the sealing member closes the opening in response to an input of the force from the transfer mechanism.

2. The cell culture container according to claim 1, comprising a cover fixable to the container, wherein
the sealing member is an O-ring that covers the opening,
the transfer mechanism has
a tapered portion that is formed at the attachment/detachment portion and that holds the O-ring;
an loop-shaped member; and
a pressure application portion that is provided at the cover and that presses the loop-shaped member against the O-ring when the cover is fixed to the container.

3. The cell culture container according to claim 1, comprising a first cover fixable to the container, wherein
the sealing member is an O-ring that covers the opening, and
the transfer mechanism has a pressure application portion that is provided at the first cover and that is pressed against the O-ring when the first cover is fixed to the container.

4. The cell culture container according to claim 3, wherein the container is provided with a through hole that communicates the second inner space with outside of the container.

5. The cell culture container according to claim 4, wherein the first cover has an elastic member that is disposed inside the first cover and that closes the through hole when the first cover is fixed to the container.

6. The cell culture container according to claim 5, further comprising:
a second cover fixable to the container; and
a screw mechanism, wherein
the screw mechanism is provided with a thread and a thread groove, the thread being formed in one of an outer side of the container and an inner side of the second cover, the thread groove being formed in the other of the outer side of the container and the inner side of the second cover, the thread being screwed into the thread groove, the second cover being fixed to the container by rotating the second cover relative to the container around a rotation axis that is along a direction orthogonal to a main surface of the membrane.

7. The cell culture device according to claim 6, wherein a recess is formed in an upper surface of the second cover.

8. The cell culture container according to claim 1, wherein
the insert member has a tubular portion and a flange portion, the tubular portion having a lower end closed by the membrane, the flange portion protruding from an outer peripheral surface of the tubular portion at an upper end of the tubular portion,
the container has a bottom wall, a side wall, and an upper wall that define the second inner space, and
the upper wall includes a standing wall protruding from the upper wall to support the flange portion.

9. A cell culture device comprising:
the cell culture container according to claim 1; and
a medium management unit that suctions and discharges a medium accommodated in at least one of the anaerobic chamber and the aerobic chamber, wherein
the cell seeded on the membrane is an intestinal epithelial cell.
